# EUROPEAN PATENT SPECIFICATION

(11) **EP 3 223 702 B1**
(45) Date of publication and mention of the grant of the patent: **11.01.2023**
(21) Application number: 15816373.3
(22) Date of filing: 24.11.2015
(51) Int. Cl.: A61B 5/08, G01N 27/22

(54) **MINIATURIZED DEVICE FOR DETERMINING A BREATHING PATTERN**
MINIATURISIERTE VORRICHTUNG ZUR BESTIMMUNG EINES ATMUNGSMUSTERS
DISPOSITIF MINIATURISÉ PERMETTANT DE DÉTERMINER UN MOTIF DE RESPIRATION

(30) Priority: 24.11.2014 EP 14194610
(43) Date of publication of application: 04.10.2017
(73) Proprietor: Université catholique de Louvain, 1348 Louvain-la-Neuve (BE)
(72) Inventor: ANDRÉ, Nicolas, 1348 Louvain-la-Neuve (BE); FLANDRE, Denis, 1170 Watermael-Boitsfort (BE); FRANCIS, Laurent, 1390 Grez-Doiceau (BE); GÉRARD, Pierre, 1348 Louvain-la-Neuve (BE); GOSSET, Geoffroy, 4000 Liège (BE); WALEWYNS, Thomas, 5336 Courrière (BE)
(74) Representative: Icosa
(86) International application number: PCT/EP2015/077527
(87) International publication number: WO 2016/083391

(56) References cited:
- US-A1- 2010 175 699
- US-A1- 2010 307 238
- US-A1- 2012 136 268
- NICOLAS ANDRE ET AL: "Dew-Based Wireless Mini Module for Respiratory Rate Monitoring", IEEE SENSORS JOURNAL, IEEE SERVICE CENTER, NEW YORK, NY, US, vol. 12, no. 3, 1 March 2012 (2012-03-01), pages 699-706, XP011408153, ISSN: 1530-437X, DOI: 10.1109/JSEN.2011.2161668 cited in the application
- PACZESNY D ET AL: "Construction of fast dew point hygrometer with integrated semiconductor detector for medical applications", RESEARCH IN MICROELECTRONICS AND ELECTRONICS, 2005 PHD JULY, 25, 2005, PISCATAWAY, NJ, USA,IEEE, PISCATAWAY, NJ, USA, vol. 2, 1 July 2005 (2005-07-01), pages 143-146, XP010859028, DOI: 10.1109/RME.2005.1542957 ISBN: 978-0-7803-9345-5
- ANDRE N ET AL: "Miniaturized Wireless Sensing System for Real-Time Breath Activity Recording", IEEE SENSORS JOURNAL, IEEE SERVICE CENTER, NEW YORK, NY, US, vol. 10, no. 1, 1 January 2010 (2010-01-01), pages 178-184, XP011286527, ISSN: 1530-437X, DOI: 10.1109/JSEN.2009.2035666 cited in the application

## Description

### FIELD OF INVENTION

The present invention concerns a device for determining a breathing pattern, and preferably the exhalation pattern of a subject, preferably a human. The present invention in particular provides a device allowing the respiratory functions of a subject to be monitored in a real-time fashion, while also permitting to detect abnormal breathing events such as apnea or dyspnea.

### BACKGROUND OF INVENTION

Respiratory monitoring is a widespread medical procedure which finds applications in many domains, from the monitoring of patients undergoing a surgery or suffering from cardio-respiratory diseases, to medical rescue or even sports. Among the three major categories of non-invasive respiratory monitoring procedures that could be used (i.e. chest movement study, airflow sensing or blood gas measurements), airflow sensing is probably the more practicable and easier procedure to be implemented to a patient. Further, airflow sensing allows measuring several parameters such as temperature, humidity or CO₂ concentration of the inspired and exhaled air. Despite the fact that respiratory monitoring has long been used for medical applications, there is still a growing demand for cheap devices which enable the monitoring of chosen human life functions in patients, including respiratory ones. Further, there is a need for new measurement methods and new breath monitoring sensors enabling to reduce the manufacturing costs, while at the same time providing more detailed information as to the immediate clinical picture of the patients.

Most of known devices for monitoring respiratory functions are capable of analyzing the frequency and the amplitude of respiration, but remain unable to provide real-time detailed information as to the presence of irregular patterns in the various phases of inhalation and exhalation. Further, there is a need for devices allowing to determine a breathing pattern with a reduced inertia, and allowing instant or immediate detection in changes in breath dynamics. Indeed, the respiratory rate of an adult amounts to 10-20 breaths per minutes (BPM), and the known-to-date sensor devices are unable to accurately measure the various breathing parameters for each breath.

WO2008/122806, WO2008/009980, EP2548505 and US2012/01656693 disclose sensors for detecting water vapor in an exhaled breath. Nevertheless, these sensors are based on metallic electrodes connected together by a porous hydro-sensitive layer. The use of a porous material renders difficult to reach subsecond response times since these materials are enduring a two-step desorption cycle: first the water layer evaporates, then the porous material desorbs. Moreover, porous structures are more sensitive to surface contamination.

Other respiratory monitoring systems of the art rely on capacitive transducers formed by planar metallic electrodes. These sensors, based on the detection of the micro-condensation of the water contained in the breathed air, display a adsorption/desorption time and are capable to operate in conditions ranging from 50% relative humidity to strong condensation (André et al., IEEE J. Sens., vol. 10 :178-184, 2010 and André et al., IEEE J. Sens., vol. 12 : 699-706, 2012). Nevertheless, these sensors are very sensitive to water condensation, a drawback that may at least in part result from an insufficient surface area of their transducer and from the use of a limited physical interface. The condensation of the water vapor contained in the breathing air streams on the surface of the sensor element is rapid and do not allow a breathing pattern to be determined. Moreover, the response times of the disclosed transducers are important and do not allow a breathing pattern to be determined in a real-time fashion, especially in wide environmental conditions. Said sensors enable to determine a respiratory rate which differs from a full breathing pattern including both the breathing envelope and flux.

Similarly, Janik et al. disclose a device for monitoring human breathing, comprising a micro-condensation sensor formed of interdigitated electrodes, wherein said sensor responds to changes in humidity in the form of changes in both capacitance and conductance, even in absence of air flow on the surface. This document further discloses that the presence of a layer allowing water to be absorbed, such as an antiseptic layer, strongly enhances the capacity of the sensor to detect micro-condensation under conditions wherein relative humidity is below 80% (Janik et al., Sens. Act. A, vol. 185 : 160-167, 2012). The disclosed sensor does nevertheless not allow a breathing pattern, and more specifically an exhalation pattern to be determined in real-time conditions, with a resolving detection of both the very first and very last instants of the exhalation phase.

### DESCRIPTION

The device of the present invention overcomes the above-mentioned drawbacks of the prior art. Indeed, the device of the present invention aims at detecting the deposition of water droplets contained in a breath air stream, and more specifically in an air stream exhaled by a subject, through the combination of an improved sensor element comprising a capacitive transducer, arranged according to a specific angle with respect to the air stream, and of a physical interface possessing a high resolution and an elevated sampling interval frequency. The device of the invention is a simple and direct measurement tool providing a reliable, portable and non-intrusive measurement of the respiratory rate.

The device of the invention thus provides a reliable non-intrusive, quantitative and costsaving solution to a long-felt need. It may be particularly useful for sleep disorder monitoring (especially considering sleep apnea, through apnea and hypopnea detection), as well as to prevent limitation of the revalidation of elderly people due to respiratory impairment, such as for example dyspnea. The device of the invention also presents the sensitivity and miniaturization features required for babies and small kids.

In one embodiment, the device of the invention is wireless.

The present invention thus concerns a method and a device for identifying breathing alterations representative of respiratory troubles or conditions.

The method for identifying breathing alterations representative of respiratory troubles or conditions, comprises:
(a) providing a device comprising:
   (i) two or three sensor elements, each composed of:
      - an isolating support formed by a dielectric material; and
      - a single capacitive transducer formed by interdigitated electrodes,
         wherein the finger width (3) of said interdigitated electrodes ranges from about 0.5 to about 10 µm;
         wherein the interspace (2) extending between the fingers of said interdigitated electrodes has a width (3) ranging from about 0.5 to about 10 µm;
         wherein the capacitive transducer has a surface area (7) of at least 4 mm²;
         wherein the materials of the electrodes or of the isolating support do not absorb water molecules;
   (ii) a physical interface suitable for transducing a capacitive signal obtained from the sensor elements into an analog or digital signal;
      wherein said physical interface has a sampling interval frequency of less than 500 Hz, or less than 200 Hz, or about or less than 100 Hz, and
      wherein said interface has a resolution of about or less than 0.1 pF, preferably about or less than 0.01 pF;
(b) placing the device so that a surface (7) of the sensor elements comprising said capacitive transducer is exposed to an exhaled air stream and forms an angle, in a sagittal plane, comprised between about -30° and about +30° with the said exhaled air stream;
   wherein the sensor elements and the physical interface are configured for determining a respiratory rate and breathing envelope; and
wherein the sensor elements are configured to discriminate and/or count water droplets suspended in said exhaled air stream as they deposit on the surface of the capacitive transducer.

The device for implementing the method of the invention comprises:
(a) two or three sensor elements, each comprising:
   (i) an isolating support formed by a dielectric material; and
   (ii) a single capacitive transducer formed by interdigitated electrodes having fingers, wherein the finger width of said interdigitated electrodes ranges from about 0.5 to about 10 µm; wherein the interspace extending between the fingers of said interdigitated electrodes has a width ranging from about 0.5 to about 10 µm, wherein the capacitive transducer has a surface area of at least 4 mm², and wherein the materials of the electrodes or of the isolating support do not absorb water molecules;
(b) at least one physical interface transducing the capacitive signal into an analog or digital signal, wherein said physical interface has a sampling interval frequency of less than 500 Hz, preferably less than 200 Hz, more preferably about or less than 100 Hz and a resolution of less than 0.1 pF, preferably less than 0.01 pF; and
wherein a surface of the sensor elements is exposed to an air stream and forms an angle comprised between about -30° and about +30° with the said air stream, wherein the sensor elements and the physical interface are configured for determining a respiratory rate and breathing envelope, and wherein the sensor elements are configured to discriminate and/or count water droplets suspended in said exhaled air stream as they deposit on the surface of the capacitive transducer.

In one embodiment, the sensor elements and the physical interface are configured for determining a breathing pattern.

In a preferred embodiment, a surface of the sensor elements is exposed to an air stream and forms, in a sagittal plane, an angle comprised between about -30° and about +30° with the said air stream.

In one embodiment, a surface (7) of the sensor elements each comprising said capacitive transducer is exposed to an exhaled air stream and also forms an angle, in a coronal plane, comprised between about -30° and about +30° with the said exhaled air stream.

In this invention, the term "about" preceding a figure means plus or less 10% of the value of said figure.

In a particular embodiment, the capacitive transducer has a surface area (7) of at least 9 mm².

In a particular embodiment, the device according to the present invention further comprises:
(a) at least one temperature sensor element, disposed for measuring the temperature of said surface of said sensor element exposed to the said air stream; and
(b) means for heating and/or cooling said surface.

In a particular embodiment, at least one temperature sensor element of the device according to the invention is selected in the group consisting of a resistor, a diode or a thermocouple.

In a particular embodiment, the interdigitated electrodes of the device according to the present invention comprise a metal selected in the group comprising Al, Ni, Au, Ag, Cu, Pt, Pd, Co and Fe or alloys thereof.

In a particular embodiment, the width of said fingers of said interdigitated electrodes ranges from 1 to 5 µm and is preferably of about 2 µm. The interdigitated electrodes used in the invention may be referred to as interdigitated microelectrodes.

In a particular embodiment, the width of said interspaces extending between the fingers of said interdigitated electrodes ranges from 1 to 5 µm and is preferably of about 2 µm.

In a particular embodiment, the dielectric material of the said isolating support is selected in the group consisting of ceramic, glass, polymers or the like, and is preferably silicon oxide.

In a particular embodiment, the said physical interface of the device according to the invention is selected in the group comprising a Schmitt trigger, a Ring oscillator, a Sigma-delta analog-to-digital converter (ADC), a C-2C Analog-to digital converter and a constant DC charge current scheme for capacitance measurement.

In a particular embodiment, the said physical interface of the device according to the invention has a sampling interval frequency of less than 500 Hz, or less than 200 Hz, or about or less than 100 Hz. Preferably the interval frequency is about 100 Hz (period is 10 ms).

In a particular embodiment, the said physical interface of the device according to the invention has a resolution of about or less than 1 fF.

In a particular embodiment, the surface area of said capacitive transducer of the device according to the invention is equal or greater than 16 mm², and preferably equal or greater than 25 mm².

In a particular embodiment, the device according to the invention further comprises:
- control means for receiving an input analog or digital signal from the said physical interface and for providing data; and
- means for recording and/or transmitting and/or displaying data generated by the said control means.

The present invention further comprises a device as described hereinabove for use for establishing the breathing pattern of a subject.

In a particular embodiment, the said device is for use for detecting abnormal patterns in the respiratory functions of a subject. In a particular embodiment, the said subject is a mammal, and preferably a human.

### BRIEF DESCRIPTION OF THE DRAWINGS

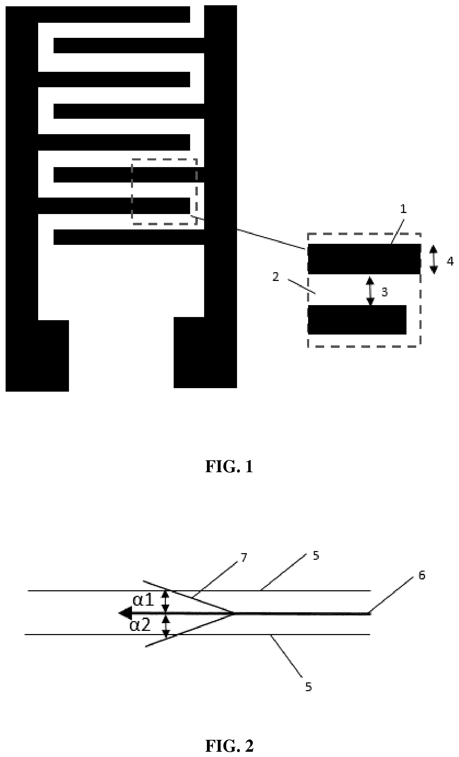

**Figure 1** is a drawing showing the schematic representation of a capacitive transducer for use in the present invention and comprising interdigitated electrodes. (1) corresponds to an electrode finger. (2) corresponds to an interspace. (3) corresponds to the width of an electrode interspace. (4) corresponds to the width of an electrode finger.
**Figure 2** is a schematic representation of the sagittal section of an air stream circulating in a tube, such as for instance a cannula. (5) corresponds to the walls of the tube. (6) corresponds to the average mean directional air stream jet in the tube. (7) corresponds to the surface of the sensor element which is to be exposed to the air stream. (α1) and (α2) correspond to the maximum side angles, with respect to the average mean directional air stream, that can be used for positioning the surface of the sensor element.

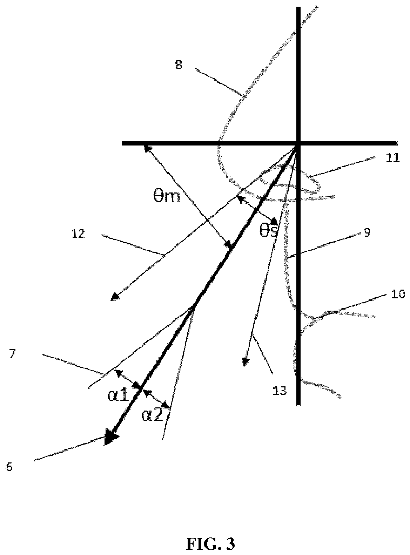

**Figure 3** is a schematic representation of a sagittal section of a nasal air stream. (8) corresponds to the nose. (9) corresponds to the philtrum. (10) corresponds to the opening of mouth. (11) corresponds to a nostril. (6) corresponds to the average mean directional nasal air stream jet. (7) corresponds to the surface of the sensor element which is to be exposed to the air stream. (α1) and (α2) correspond to the maximum side angles, with respect to the average mean directional nasal air stream, that can be used for positioning the surface of the sensor element. (12) corresponds to the maximum upper limit of the directional nasal air stream. (13) corresponds to the maximum lower limit of the directional nasal air stream. (θm) corresponds to the average mean side angle of the directional nasal air stream jet. (θs) corresponds to the spreading angle of the nasal air stream.

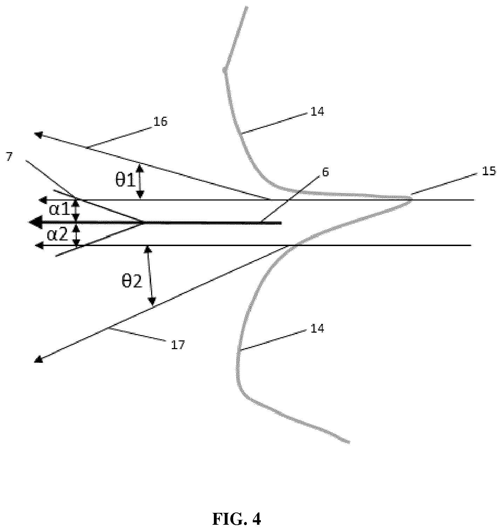

**Figure 4** is a schematic representation of a sagittal section of a mouth air stream. (14) corresponds to the upper and lower lips. (15) corresponds to the mouth opening. (6) corresponds to the average mean directional mouth air stream jet. (7) corresponds to the surface of the sensor element which is to be exposed to the air stream. (α1) and (α2) correspond to the maximum side angles, with respect to the average mean directional mouth air stream, that can be used for positioning the surface of the sensor element. (16) corresponds to the maximum upper directional limit of the mouth air stream. (17) corresponds to the maximum lower directional limit of the mouth air stream. (θ1) and (θ2) correspond to the side spreading angles of the mouth air stream.

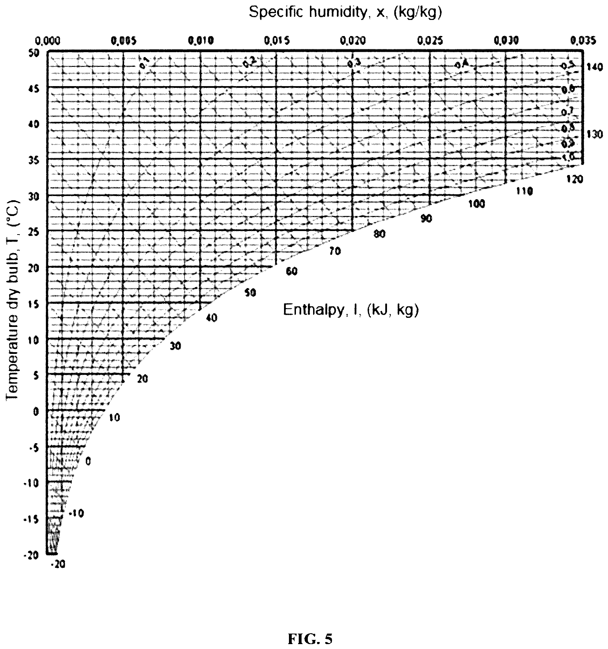

**Figure 5** corresponds to a diagram of Mollier representing the dry bulb temperature (y axis, expressed in °C) as a function of the specific humidity (x axis, expressed in kg/kg) as a function of the enthalpy (curve, expressed in kJ.kg).

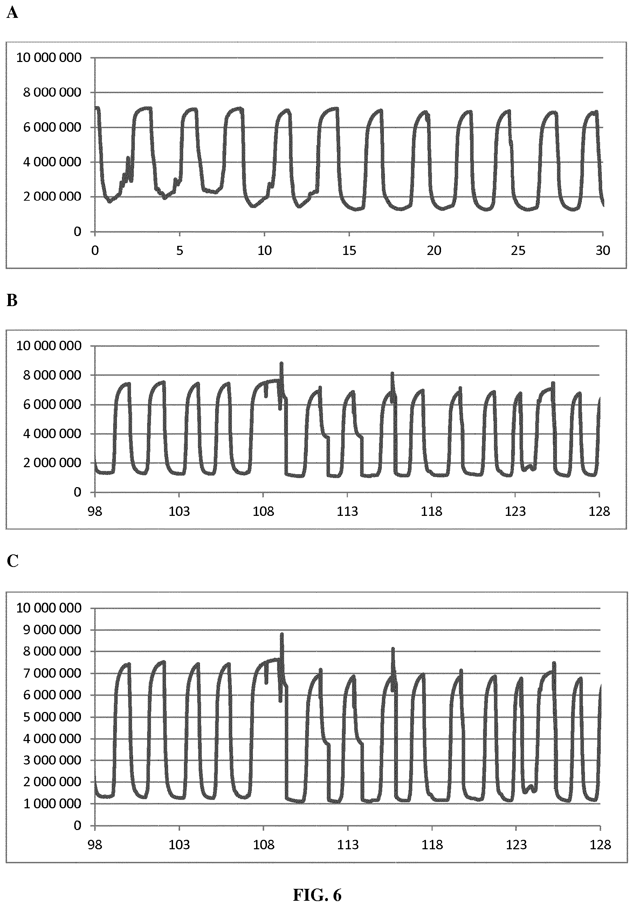

**Figure 6** corresponds to a graph representing the water vapor content in the nasal air stream of a subject as a function of time (x axis, in seconds). Such graphs are also known as capnograms. Figure 6A corresponds to the capnogram obtained from a healthy patient at rest. Figure 6B corresponds to the 30 last seconds of the subject breathing after having walked for 5 minutes. Figure 6C corresponds to the capnogram of the subject recovering from the walk. Within the meaning of the present invention, by "breathing pattern", it is meant whole or part of the key parameters characterizing a breathing. The expression breathing pattern may thus more specifically encompass parameters such as the respiratory frequency, the breathing rate, the inspiratory frequency, the expiratory frequency, as well as the duration of inhalation and/or of exhalation phases. In a particular embodiment of the invention, the device according to the present invention allows measuring directly and precisely the duration and frequency of the exhalation phase. In a particular embodiment of the invention, the expression "breathing pattern" may also encompass the scheme representing the breathing over several inhalation/exhalation cycles.

### DETAILED DESCRIPTION

This invention thus relates to a method according to claims 1 to 14 and a device according to claim 15.

The sensor elements and the physical interface of the device of the invention are configured for determining a full breathing pattern.

Within the meaning of the present invention, by "sensor element", it is meant a sensor discriminating and/or counting the water droplets that are suspended in an air stream. The water droplets are detected as they are deposited on a surface (7) of the said sensor element placed into contact with an air stream. In a particular embodiment, the said air stream corresponds to a breathed air stream, preferably to an inhaled and/or exhaled air stream and more preferably to an exhaled air stream. In a particular embodiment, the said air stream corresponds to a nasal and/or oral air stream. In a further particular embodiment, the said air stream corresponds to an air stream inhaled and/or exhaled by one nostril, by both nostrils, by mouth and/or by a combination thereof. In a particular embodiment, the said breathed air stream is generated by a mammal, preferably a human or an animal.

In a preferred embodiment, the water sensor element according to the invention has a minimal detection capacity of at least 10 deposited water droplets, preferably of at least 15 deposited water droplets. In a particular embodiment, the minimal detection capacity of the water sensor element according to the invention is of about 20 deposited water droplets.

In a particular embodiment of the invention, the device of the invention comprises two or three sensor elements. In a particular embodiment, the said device comprises two sensor elements, which are preferably disposed such that each sensor element is exposed to the air stream flowing through one nostril. Advantageously, a device according to the invention comprising two sensor elements allows the air stream of both nostrils to be analyzed simultaneously and independently. In another particular embodiment, the device according to the invention allows the water droplet content of the air stream inhaled and/or exhaled by both nostrils to be determined and analyzed, while simultaneously tolerating a normal mouth breathing of the subject. In another embodiment of the present invention, the said device comprises one sensor element, which is preferably disposed such as to be exposed to the air stream inhaled and/or exhaled by the mouth. In another embodiment, the device of the invention comprises three sensor elements, wherein two sensors elements allow the air stream of each nostril to be analyzed independently, and the third sensor allows the air stream of the mouth to be analyzed.

The sensor element according to the invention comprises a capacitive transducer formed by interdigitated electrodes. Within the meaning of the present invention, by "capacitive transducer", it is meant an electrical system wherein an electrical charge is applied between two conductive objects (hereinafter the "electrodes"), separated one from each other by a gap (hereinafter "the interspace"). The capacitive transducer for use in the present invention is capable to sense its environment in the form of capacitor changes. The said capacitive transducer thus allows a water droplet placed between the electrodes to be detected. The capacitive transducer for use in the invention more specifically comprises a plurality of electrode portions arranged in the form of an array of interdigitated electrode portions, for instance arranged in a concentric or rectangular pattern. Such capacitive transducers are well known to the skilled person in the art and are also referred to as interdigitated electrodes (or "ITE"). Within the meaning of the invention, the term "fingers" refers to the isolated portions of electrodes that are interdigitated.

The electrodes for use in the capacitive transducer according to the invention may be formed by any suitable technique. In one embodiment, the technique comprises printing the electrode material in the form of a thick film screen printing ink onto an isolating support. Alternative techniques for preparing the electrodes and applying them to an isolating support include spin/sputter coating and visible/ultraviolet/laser photolithography. In order to avoid impurities being present in the electrodes, which may alter the electrochemical performance of the sensor element, the electrodes may also be prepared by electrochemical plating ("electroplating"). In particular, each electrode may comprise a plurality of layers applied by different techniques.

The electrodes for use in a capacitive transducer as shown in Figure 1 may be formed of any material suitable for capacitive transduction. By "material suitable for capacitive transduction" it is meant that the said electrodes are formed in a material allowing an electric, and more specifically a capacitive, transduction to be formed, more specifically upon linking both electrodes by water droplets.

In a particular embodiment, materials suitable for capacitive transduction according to the present invention include metallic materials, and are preferably selected in the group consisting in Al, Ni, Au, Ag, Cu, Pt, Pd, Co, Fe or alloys or combinations thereof. In a preferred embodiment of the invention, the electrodes of the capacitive transducer are formed of Al or alloys thereof.

The thickness of the electrodes for use in capacitive transducer according to the present invention is determined by the manufacturing technology, but has no direct influence on the capacitive detection. In a particular embodiment, the thickness of the electrodes ranges from 100 nm to 900 nm, and is preferably of about 500 nm.

The capacitive transducer according to the present invention is such that the width (4) of the electrode fingers forming the interdigitated electrode ranges from 0.5 to 10 µm, and is preferably of about 2 µm.

Further, the capacitive transducer according to the present invention is also such that the width (3) of the interspaces extending between the electrode fingers forming the interdigitated electrode ranges from 0.5 to 10 µm, and is preferably of about 2 µm.

In a particular embodiment of the present invention, the width (4) of the electrode fingers (1) and the width (3) of the interspaces (2) extending between said electrode fingers (1) are substantially equivalent, and are preferably of about 2 µm.

The present inventors have indeed surprisingly discovered that such respective width (3,4) for the fingers (1) and the interspaces (2) of the interdigitated electrodes used in the device of the invention advantageously allow deposited water droplets to be detected as from a minimal deposition of at least 10, preferably at least 15 and more preferably of about 20 water droplets on the surface of the capacitive transducer. Water droplets suspended in an inhaled and/or exhaled air stream are indeed known to have a mean diameter of from about 2 µm to about 20 µm. The respective widths (3,4) of the electrode fingers (1) and interspaces (2) used in the device of the present invention is especially selected for allowing variations of the capacitive transduction to be detected between two electrode fingers as a result of water droplet deposition. Nevertheless, a reliable detection of the water droplets deposition on the surface of the capacitive transducer of the invention requires the cumulative alterations of capacitive transduction resulting from as few as at least 10, preferably at least 15, more preferably of about 20 water droplets to be detected. This detection capacity thus renders possible, for the device of the invention, to detect the onset of the exhalation phase with a very high precision, and thus contribute to the capacity of said device to acquire data relating to the full breathing envelope of a subject, and thus to identify breathing alterations that are representative of respiratory troubles or conditions.

In a particular embodiment, the portions of the electrodes that are not disposed to be in contact with the air stream (that is the non-operational portions of the electrodes) may be coated with a dielectric material, patterned in such a way as to leave exposed the active portions of the electrodes.

The sensor element according to the invention further comprises an isolating support formed by a dielectric material. In the device according to the invention, the interdigitated electrodes forming the capacitive transducer of the invention are plated onto the said isolating support. The said isolating support may thus be formed by any suitable material, provided that it does not react or interact with the electrodes or with the water droplets suspended the air stream to be analyzed. In a particular embodiment, the said isolating support is formed of a non-conductive material, for example ceramic, glass, polymers (e.g. plastics) or the like. In a particular embodiment, the said isolating support is formed of oxides, in particular metal oxides, including aluminium oxide, silicon oxide, titanium oxide, zirconium oxide and mixtures thereof. In a particular embodiment, the said isolating support is formed of a silicon oxide or a silicon dioxide. In another particular embodiment, the isolating support is made of a ceramic material such as Si₃N₄.

The capacitive transducer for use in the device of the invention has a surface area of at least 4mm². In a particular embodiment, the surface area of the capacitive transducer of the invention is equal or greater than 9 mm². In another particular embodiment of the invention, the surface area of said capacitive transducer is equal or greater than 16 mm². In another particular embodiment of the invention, the surface area of said capacitive transducer is equal or greater than 25 mm². The present inventors have indeed surprisingly discovered that a minimal surface area of 4 mm² is required for allowing the full breathing envelope to be determined, in avoiding saturating the detection capacity of the said device, and more specifically of the capacitive transducer. As discussed previously, water droplets in suspension in an air stream start to be deposited on the surface of the sensor element according to the invention when the device is exposed to the said air stream. A similar situation occurs when the device according to the invention is exposed to a starting exhalation air stream in a subject. The water droplets deposited on the surface of the capacitive transducer are being detected as from 10, preferably as from 15 and more preferably as from 20 droplets and induce a first signal variation of the capacitive transduction. This phenomenon amplifies as the exhalation progresses, since an increasing number of water droplets are depositing on the surface of the capacitive transducer. The accumulation of the deposited water droplets leads to their progressive aggregation, for forming large water drops, then for forming a water film. The water film formed at the surface of the capacitive transducer at the end of the exhalation phase generally leads to the saturation of the transduction signal in the respiratory monitoring devices known in the art. The present inventors have nevertheless surprisingly discovered that a capacitive transducer according to the present invention and having a minimal surface area of 4 mm² is not saturated by the accumulated water droplets deposited along a full exhalation phase. It therefore results that the device of the invention now renders it possible to acquire the complete information relating to the breathing envelope of a subject, and thus overcomes the drawbacks encountered with respiratory monitoring devices known in the art. Further, the surface area of the capacitive transducer used in the device of the invention is such that the water film formed at the end of the exhalation phase is fully evaporated during the subsequent inhalation phase. This property allows the device of the invention to acquire data relating to the breathing process in a real-time fashion.

The sensor element according to the invention is in particular such that the materials used for forming the electrodes of the capacitive transducer or the isolating support are not absorbing water molecules. The resolution and the dynamics of the said sensor are therefore not affected by the absorption/desorption of water, such as observed in several respiratory monitoring systems known in the art.

The device of the invention further comprises a physical interface suitable for transducing a capacitive signal obtained from the sensor elements into an analog or digital signal. In a particular embodiment, the said capacitive interface is capable to transduce the capacitance value obtained from the sensor element into a voltage, current, frequency, time or any other digital value proportional to the measured capacitance value. As a result, the electric change measured is directly linked to the response of the sensor element to fluctuations of water droplets deposition caused by the breathing of a patient to which the device of the invention is applied. Interfaces measuring the resistive behavior of electrodes are specifically excluded from being used in the device of the invention.

The physical interface for use in the device of the invention has a sampling interval frequency of less than 500 Hz, or less than 200 Hz, or about or less than 100 Hz. In a particular embodiment of the invention the said physical interface has a sampling interval period of about 50 ms or less, more preferably of about 20 ms or less, preferably of about 10 ms or less. In a particular embodiment, the sampling interval period of said physical interface ranges from 1 ms to 128 ms, preferably from 10 ms to 100 ms, more preferably from 10 ms to 50 ms. The sampling interval frequency of the physical interface for use in the device of the present invention renders it possible to acquire a large number of capacitive signals issued by the sensor element along the inhalation and exhalation phases of the breathing process. The high sampling interval frequency of the physical interface of the invention thus participates to the capacity of the device of monitoring the breathing envelope of a subject in a real-time fashion, with a very high resolution.

Further, the physical interface for use in the device of the invention has a capacitance resolution of about less than 10 pF. In a particular embodiment of the invention the said physical interface has a capacitance resolution of less than 10 pF, preferably of less than 1pF and more preferably of about 0.01 pF. In another particular embodiment of the invention, the capacitance resolution of the physical interface of the invention ranges from about 0.01 pF to about 10 pF, and preferably from about 1 pF to about 5 pF. The capacitance resolution of the physical interface for use in the device of the invention thus participates to the capacity of the said device to detect as few as from 10 deposited water droplets, preferably as few as from 15 deposited water droplets and more preferably as few as about 20 deposited water droplets on the surface or the sensor element exposed to the air stream.

It is herein highlighted that the resolution of the physical interface (the smallest change that a sensor can detect) differs from the sensibility of the physical interface (minimum change of input signal required to produce a specified output signal).

In a particular embodiment of the invention, the said physical interface is selected in the group comprising a Schmitt trigger, a Ring oscillator, a Sigma-Delta analog-to-digital converter (ADC), a C-2C Analog-to digital converter and a constant DC charge current scheme for capacitance measurement. In a preferred embodiment, the said physical interface is a constant DC charge current scheme for capacitance measurement, wherein the charging time and the value of the charge current are able to be reprogrammed during operation (i.e. "on fly"), thus allowing the dynamics of the voltage accumulated in the capacity to be optimized. In a preferred embodiment, the physical interface is a constant DC charge current scheme for capacitance measurement such as a MPR121 physical interface (Freescale Semiconductor Inc.). In an embodiment, the physical interface may have a range of capacitance of from 10 pF to 2000 pF. In an embodiment, the physical interface may have a range of capacitance of from one unit to at least 100 times this unit (for example from 1 pF to 100 pF).

Further, in a particular embodiment, the physical interface for use in the device of the invention has the capacity of being reprogrammed during the acquisition of capacitive data issued by the sensor element. The said physical interface is indeed advantageously capable of monitoring the voltage of the capacitive transducer of the sensor element and to modify, where necessary, the value of the charge/discharge current and/or the value of the charge/discharge time to ensure the best dynamic response of the sensor element. By determining a maximum and minimum value of the voltage measured on the capacitive transducer, the physical interface can dynamically modify the value of the time charge/discharge and/or the value of the charge/discharge current if electrode data goes out of valid range during the measurement, and this to ensure a maximal resolution of the sensor measurements.

The sampling interval frequency and/or period of the said physical interface can thus be modified in a real-time fashion for ensuring that the detection thresholds of capacitive variations are in adequation with the capacitive signal issued by the sensor element.

In one embodiment, the said sensor elements are directly exposed to an air stream. In one embodiment, the air stream is not directed to the sensor by means of any suction pipe.

The device of the invention is further such that the side angle between the surface of the water sensor element exposed to the said air stream and the air stream is comprised between -30° and +30°. By "side angle", it is meant the angle formed by the surface of the water sensor element, comprising the capacitive transducer, which is exposed to the said air stream and the average mean directional jet of the air stream, as visualized on a side view of the air stream, i.e. as visualized on a sagittal section thereof (see for instance Figure 2). The determination of a breathing pattern based on the deposition of the water droplets contained in the said air stream requires that at least whole or part of the sensor element be placed within the side spreading angle of the measured air stream jet (6).

In one embodiment, the surface (7) of the sensor element comprising said capacitive transducer is exposed to an exhaled air stream and forms an angle, in a sagittal plane, comprised between about -30° and about +30° with the said exhaled air stream, or between about -29° and about +29°, or between about -28° and about +28°, or between about -27° and about +27°, or between about -26° and about +26°, or between about -25° and about +25°, or between about -24° and about +24°, or between about -23° and about +23°.

Within the present invention sagittal plane refers to a vertical plane which passes from anterior to posterior, dividing the body into right and left halves.

In one embodiment, the surface (7) of the sensor element comprising said capacitive transducer is exposed to an exhaled air stream and forms an angle, in a coronal, comprised between about -30° and about +30° with the said exhaled air stream, or between about - 29° and about +29°, or between about -28° and about +28°, or between about -27° and about +27°, or between about -26° and about +26°, or between about -25° and about +25°, or between about -24° and about +24°, or between about -23° and +23°, or between about -22° and about +22°, or between about -21° and about +21°.

Within the present invention "coronal plane" refers to a vertical plane that divides the body into ventral and dorsal sections and containing the nostrils.

In a particular embodiment, when the air stream is a nasal breathing air stream, the side angle between the surface of the sensor element exposed to the said air stream and the air stream is comprised between -30° and +30°. By "side angle", it is meant the angle formed by the surface of the sensor element exposed to the said air stream and the average mean directional jet of a nostril air stream, as visualized on a sagittal section of the head (see Figure 3). In a preferred embodiment, the average mean directional jet of a nostril air stream is considered as forming a side angle of 60°± 6° with the horizontal, as shown on Figure 3 (see also Gupta et al., "Characterizing exhaled air flow from breathing and talking", Indoor Air, 2010, 20:31-39). For allowing the determination of a breathing pattern based on the deposition of the water droplets contained in the said nasal air stream, at least whole or part of the sensor element has to be placed within the side spreading angle of 23° ± 14° of the measured air stream jet (6) as shown on Figure 3.

In a particular embodiment, when the air stream is a mouth breathing air stream, the side angle between the surface of the sensor element exposed to the said air stream and the air stream is comprised between -30° and +30°. By "side angle", it is meant the angle formed by the surface of the water sensor element exposed to the said air stream and the average mean directional jet of a mouth air stream, as visualized on a sagittal section of the head (see Figure 4). In a preferred embodiment, the average mean directional jet of a mouth air stream is considered as being substantially horizontal, as shown on Figure 5 (see in particular Gupta et al., "characterizing exhaled air flow from breathing and talking", Indoor air, 2010, 20:31-39). For allowing the determination of a breathing pattern based on the deposition of the water droplets contained in the said mouth air stream, at least whole or part of the water sensor element has to be placed within the side spreading angle of 30° (corresponding to the sum of the angles θ1 and θ2 on Figure 4) of the measured mouth air stream jet (6) as shown on Figure 4.

In one embodiment, the exhaled air stream has the shape of a cone and the surface (7) of the sensor element comprising said capacitive transducer is exposed to the exhaled air stream and forms with respect to the height of the cone an aperture angle comprised between about -30° and about +30° with the said exhaled air stream, or between about - 29° and about +29°, or between about -28° and about +28°, or between about -27° and about +27°, or between about -26° and about +26°, or between about -25° and about +25°, or between about -24° and about +24°, or between about -23° and about +23°.

The device according to the present invention differs from the respiratory sensing devices known in the art at least in that it does not rely on the monitoring of the condensation of the vapored water contained in the breathed air stream. The device of the present invention does indeed contain no porous or mesoporous film of ion exchange material extending between the electrode fingers. Further, it is to be noted that within standard conditions (e.g. within external temperature conditions of from 20 to 35°C, and of from 40% to 60% relative humidity), the resolution and the dynamics of the device of the present invention are not affected or altered by the possible condensation of the water vapor contained in the breathing air streams on the surface of the sensor element. Within such operative conditions, the condensation of the water vapor contained in the breathing air streams on the surface of the sensor element is regarded as negligible when compared to the deposition of water droplets suspended in the said air streams.

Nevertheless, when the device according to the present invention is operated in conditions wherein the external residual humidity is greater than 60%, wherein the external temperature is below 20°C, or wherein the temperature of the breathing air stream is 30% greater than that of the surface of the sensor element, the water vapor contained in the exhaled air stream may condense importantly on the surface of the sensor element and may then affect the determination of capacitive variations along the exhalation phase.

Similarly, when the device according to the present invention is operated in conditions wherein the external residual humidity is lower than 40% or wherein the external temperature is above 35°C, the water droplets suspended in the exhaled air stream may evaporate rapidly after their deposition of the surface of the sensor element, thus possibly altering the determination of capacitive variations along the exhalation phase. In such conditions, there is therefore a need for that the temperature of the surface of the sensor element be maintained in a range rendering it possible to be used without being affected by the external relative humidity or temperature.

In a particular embodiment, the device according to the invention thus further comprises at least one temperature sensor element, disposed for measuring the temperature of the surface of the sensor element exposed to the air stream, and means for heating and/or cooling the surface of the water sensor element exposed to the air stream. In a particular embodiment, the said temperature sensor element may be formed of an additional electrode. Such techniques are known in the art. In a preferred embodiment, when the device according to the invention comprises two or more sensor elements, two or more temperature sensor elements may also be provided, such that the temperature of the surfaces of each sensor element is monitored independently. In a particular embodiment, the means for measuring the temperature of the surface of the sensor element exposed to the air stream are selected in the group consisting of a resistor, a diode or a thermocouple.

In a particular embodiment, the means for heating may comprise a microheater and the means for cooling may comprise a microcooler, such dispositives being known in the art.

The said means for heating and/or cooling the surface of the sensor element can thus be used to control the temperature of the surface of the sensor element exposed to the air stream such that this temperature is maintained in a range of from -10% to +10% around the dew point temperature of the water vapor contained in the said air stream. By "dew point temperature", it is meant the temperature at which the water vapor starts to condense out of the air, i.e. the temperature at which saturation is reached when the moisture of air remains constant. Above this temperature, the moisture stays in the air. In the present invention, the expressions "dew point temperature" or "condensation temperature" are used as synonyms.

The dew point temperature of the water contained in the air stream depends from the temperature of the said air stream, from the temperature of the surface of the sensor element exposed to the air stream, from the humidity content in the air stream and of the atmospheric pressure of the location where the device of the invention is operated. The dew point temperature may be measured and/or calculated by any method known in the art including enthalpy/entropy diagrams or charts, psychrometric diagrams and the like. In a particular embodiment, the dew point temperature of the water contained in the air stream is measured through the use of a Mollier diagram, preferably a Mollier diagram displaying the dry bulb temperature as a function of humidity, as a function of enthalpy. In a preferred embodiment, the Mollier diagram used is displaying the dry bulb temperature as a function of specific humidity, as a function of enthalpy, such as that represented in Figure 5.

In a particular embodiment, the device according to the invention may further comprise control means suitable for receiving an input analog or digital signal from the said physical interface. Advantageously, the control means of the device of the invention allows the electric signal associated to the capacitance variation to be set to a maximal value when the capacitance in minimal (dry surface) and a minimal value when the capacitance is maximal (when the deposition of water droplets on the surface of the sensor element is maximal, i.e. when at the end of exhalation phase). Thereby, the measurement range is maximal leading to an optimal display of the breathing envelope, facilitating the visualization of small nasal flows volume. In a particular embodiment, the said control means are not physically localized with the sensor element and can be positioned remotedly.

In a particular embodiment, the device according to the invention further comprises means for recording and/or transmitting and/or displaying data received by the control means. In a preferred embodiment, the device of the invention comprises means for transmitting the data, such as for instance a radio emitter capable to transmit data to a suitable receiver by radio. In a preferred embodiment, the said means for transmitting data include wireless data transfer means, and more particularly transmitting means that are compatible with low energy communication transmission protocols standards ZigBee^{®} or Bluetooth^{®}. In an embodiment, the said means for transmitting data include USB interfaces such as micro USB interfaces. In an embodiment, the device of the invention comprises means for recording electronical data, such as flash memory or microSD.

In an embodiment, the device of the invention further comprises means for displaying the data corresponding to the variations of water droplets deposition measured in the course of the breathing. Such displaying means may for instance include an oscilloscope, light-emitting diodes (LEDs) or any other suitable displaying mean. The variations of water droplets deposition on the surface of the sensor element, for instance during the inhalation and/or exhalation cycles may thus be directly visualized in real-time, for instance with brightness intensity varying according to the output sensing signal.

Advantageously, the device of the invention comprises both recording and transmission means and further optionally comprises displaying means. In a particular embodiment, the device of the invention may further comprise light-emitting diodes (LEDs) or the like for system calibration and/or visual monitoring.

In a particular embodiment, the said control means also receive input data from the said temperature sensor element(s), and send input data to the means for heating and/or cooling. The control means thus allows data generated by the sensor element and the temperature sensor element to be collected such as to generate the heating or the cooling of the sensor area for allowing the temperature of the surface of the sensor element to be maintained within a suitable range for allowing the determination of the breathing pattern to be operated adequately.

The device according to the invention may further comprise a housing element comprising said sensor element, and disposed such that the angle between the surface of the sensor element to be exposed to the air stream and the air stream is comprised within the hereinabove listed side angle values. The housing element for use in the device of the invention is indeed such that the orientation of the surface of the sensor element is defined in order to optimize both water droplet deposition during the exhalation phase and forced drying of the capacitive transducer surface during the inhalation phase. In a particular embodiment, when the air stream is a nasal air stream, the housing element is such that the angle between the surface of the sensor element to be exposed to the air stream and the philtrum of the subject (i.e. the vertical groove in the middle area of the upper lip, common to many mammals, extending from the nose (8) to the upper lip (14)) is comprised between 0° and 60°, preferably between 10° and 50°, preferably between 20° and 40°, preferably between 25° and 35°; and is more preferably of 30°.

In an particular embodiment, the housing element also comprises said at least one temperature sensor element, said means for heating and/or cooling, said physical interface, said control means and said means for storing and/or transmitting and/or displaying data.

In a particular embodiment, the housing element of the device of the invention is mountable on the upper lip of a subject. In a particular embodiment, the said housing comprises apertures allowing the sensor element to be in direct contact with the air stream of the subject. In a particular embodiment, the said housing element may further comprise an open ended conduit extending longitudinally between the mouth and the nose of a subject, or under the mouth of a subject, such that the nasal or mouth air stream of the subject is channeled to a surface of the water sensor element. In a particular embodiment the device of the present invention is compatible with a nasal *cannulae.* In another particular embodiment, the said housing element is mountable on a tubular conduit or a cannulae connected to the mouth and/or to the nostrils of the subject, and allows the surface of the sensor element to be exposed to the air stream circulating inside the conduit/cannulae to be positioned according to the side angle requirements discussed above.

The precise form of the housing, or of the conduit, is not critical to the operation or performance of the device and may take any desired form. It is preferred that the body or housing of the device is prepared from a non-conductive material, such as a suitable plastic.

The device according to the invention may further comprise a power supply. In a particular embodiment, the power supply is a battery.

The device of the invention thus allows a breathing pattern to be determined, and more specifically directly allows the duration and the frequency of the exhalation phase to be determined in a real-time fashion. The said device of the invention may thus be used for acquiring a real-time breathing frequency or duration of a subject during a physical exercise or at rest. Preferably, the device of the invention is useful in domains such as elderly rehabilitation, sudden infant death monitoring, sport training and sleep disorder early diagnosis and/or monitoring. In a particular embodiment, the device of the invention allows pathological breathing patterns to be identified. Such pathological breathing patterns include more particularly bradypnea, characterized by slow respirations, tachypnea, characterized by a respiratory rate increase, hyperventilation, characterized by an increase in respiratory rate or tidal volume, or both, Kussmaul respirations, characterized by deep respirations with fast, normal or slow rate, Cheyne-Stokes, characterized by waxing and waning with periods of apnea between a repetitive pattern, apneusis, characterized by sustained maximal inhalation with pause, Biot's respiration, characterized by irregular periods of apnea alternating with periods in which four or five breaths of identical depth are taken, or ataxic patterns, characterized by irregular and unpredictable pattern with periods of apnea.

The device according to the invention does nevertheless not allow to quantify the volumes of air inhaled and/or exhaled and could therefore not be implemented for determining breathing volumes.

As a result, the device of the invention allows for a real-time display of the breathing frequency of people during a physical exercise or at rest. Preferably, the device of the invention is useful in domains such as elderly rehabilitation, sudden infant death monitoring, sport training and sleep disorder early diagnosis and/or monitoring.

The device according to the invention may thus be implemented for monitoring and determining the respiratory rate and envelope of breathing of a subject, in particular a mammal, such as a human subject or an animal. The device is particularly useful for applications requiring fast response times, for example personal respiratory monitoring of patients undergoing surgery. The device according to the present invention may further be generally used in the field of respiratory medicine, airway diseases, both restrictive and obstructive, airway tract disease management, and airway inflammation. The present invention finds particular application in the field of monitoring and management of airway diseases (such as asthma). In particular, due to the versatility of the method and speed of response that may be achieved using the device of the present invention, the results may be used to provide an early alert to the onset of respiratory diseases or illness.

The device according to the invention is advantageously easily fabricated from low-cost materials and is adaptable for use in various medical environments, and for the monitoring of various medical conditions. The device of the invention is particularly suitable for use in hospital and primary healthcare, especially for critical patients. The device of the invention further finds its use in the monitoring of a subject suffering from respiratory impairment, especially sleep apnea.

The ability of measuring the full breathing envelope in the exhaled air stream of a subject or animal may also be used in the diagnosis of Adult Respiratory Distress Syndrome (ARDS), and end-stage life-threatening lung disease. The device according to the invention is particularly useful for applications requiring fast response times (e.g. requiring subsecond response times), for example personal respiratory monitoring of tidal breathing (capnography). Capnographic measurements can be applied generally in the field of respiratory medicine, airway diseases, both restrictive and obstructive, airway tract disease management, where the shape of the capnogram changes as a function of the extent of the disease.

In one embodiment, the device of the invention is used simultaneously or sequentially with a means for measuring cardiac rhythm, arterial pressure and/or SpO2.

### EXAMPLES

The present invention is further illustrated by the following example.

### Example 1

A device having the general configuration shown in Figures 1-3 was prepared for measuring the nasal breathing of healthy subjects and of subjects suffering from respiratory troubles. The device used for these experiments comprised 2 sensor elements, wherein the electrodes are in Aluminum and the isolating support is made of silicon oxide. The width (4) of the fingers of the electrodes is of 2 µm and the width (3) of the interspace (2) between the electrodes is of 2 µm. Each sensor element for use in the said device had a surface area (7) of 9 mm².

The said sensor elements were placed in a housing designed for monitoring the nasal breathing of human subjects such that each sensor element is placed in the air stream of one nostril. The said sensor elements were operatively connected to a physical interface MPR121 (Freescale Semiconductor Inc.).

The side angle formed between each average mean directional nostril air stream and the surface (7) of each sensor element exposed to the air stream was of about 0° (i.e. the surface (7) of each sensor element was substantially parallel to the average mean nostril air stream). The device further comprised one physical interface, as well as control means and transmitting means.

The tests were conducted under regular atmospheric pressure (i.e. about 1013.25 hPa), at room temperature (i.e. at about 20°C). The nasal air stream of the tested subjects was shown to have a temperature of about 34-36°C and a relative humidity of about 50-60%. The breathing of the subjects was measured at rest, then they were instructed to have a walk for 5 to 15 minutes. After the walk, the subjects were requested to rest again and the recovery parameters were also measured.

The collected data demonstrate that the envelope and flux of the breathing could be measured in real-time, during the physical exercise as well as at rest. The collected data were processed by a computer and the corresponding capnograms were prepared (see Figure 6 A, B and C). As could be seen in the capnograms of Figure 6, the general pattern expected of a healthy subject is obtained with the device of the invention. The resulting capnograms further demonstrate that not only the device of the invention allows the breathing envelope of the subject to be measured in real-time, but it also allows data to be collected without observing any saturation of the sensor element.

## Claims

1. A method for identifying breathing alterations representative of respiratory troubles or conditions, comprising:
(a) providing a device comprising:
(i) two or three sensor elements, each composed of:
- an isolating support formed by a dielectric material; and
- a single capacitive transducer formed by interdigitated electrodes, wherein the finger width (3) of said interdigitated electrodes ranges from about 0.5 to about 10 µm;
wherein the interspace (2) extending between the fingers of said interdigitated electrodes has a width (3) ranging from about 0.5 to about 10 µm;
wherein the capacitive transducer has a surface area (7) of at least 4 mm²; wherein the materials of the electrodes or of the isolating support do not absorb water molecules;
(ii) a physical interface suitable for transducing a capacitive signal obtained from the sensor elements into an analog or digital signal;
wherein said physical interface has a sampling interval frequency of less than 500 Hz, or less than 200 Hz, or about or less than 100 Hz, and
wherein said interface has a resolution of about or less than 0.1 pF, preferably about or less than 0.01 pF;
(b) placing the device so that a surface (7) of the sensor elements comprising said capacitive transducer is exposed to an exhaled air stream and forms an angle, in a sagittal plane, comprised between about -30° and about +30° with the said exhaled air stream;
wherein the sensor elements and the physical interface are configured for determining a respiratory rate and breathing envelope; and
wherein the sensor elements are configured to discriminate and/or count water droplets suspended in said exhaled air stream as they deposit on the surface of the capacitive transducer.

2. The method according to claim **1,** wherein the capacitive transducer has a surface area (7) of at least 9 mm².

3. The method according to claim **1** or **2,** wherein a surface (7) of the sensor elements comprising said capacitive transducer is exposed to an exhaled air stream and forms an angle, in a coronal plane, comprised between about -30° and about +30° with the said exhaled air stream.

4. The method according to any one of claims **1** to **3,** wherein the device further comprises:
(a) at least one temperature sensor element, disposed for measuring the temperature of said surface (7) of each sensor element exposed to the said air stream; and
(b) means for heating and/or cooling said surface (7).

5. The method according to claim **4,** wherein the at least one temperature sensor element is selected in the group consisting of a resistor, a diode or a thermocouple.

6. The method according to any one of claims **1** to **5,** wherein said interdigitated electrodes comprise a metal selected in the group comprising Al, Ni, Au, Ag, Cu, Pt, Pd, Co and Fe or alloys thereof.

7. The method according to any one of claims **1** to **6,** wherein the width of said fingers (1) of said interdigitated electrodes ranges from 1 to 5 µm and is preferably of about 2 µm.

8. The method according to any one of claims **1** to **7,** wherein the width (3) of said interspaces (2) extending between the fingers (1) of said interdigitated electrodes ranges from 1 to 5 µm and is preferably of about 2 µm.

9. The method according to any one of claims **1** to **8,** wherein the dielectric material of the said isolating support is selected in the group consisting of ceramic, glass, polymers or the like, and is preferably silicon oxide.

10. The method according to any one of claims **1** to **9,** wherein the said physical interface is selected in the group comprising a Schmitt trigger, a Ring oscillator, a Sigma-delta analog-to-digital converter (ADC), a C-2C Analog-to digital converter and a constant DC charge current scheme for capacitance measurement.

11. The method according to any one of claims **1** to **10,** wherein the said physical interface has a sampling interval period of less than 50 ms, more preferably of less than 20 ms, and preferably of about 10 ms.

12. The method according to any one of claims **1** to **11,** wherein the said physical interface has a resolution of about or less than 1 fF.

13. The method according to any one of claims **1** to **12,** wherein the surface area (7) of said capacitive transducer is equal or greater than 16 mm², and preferably equal or greater than 25 mm².

14. The method according to any one of claims **1** to **13,** wherein the device further comprises:
- control means for receiving an input analog or digital signal from the said physical interface and for providing data; and
- means for recording and/or transmitting and/or displaying data generated by the said control means.

15. A device for implementing the method according to any one of claims **1** to **14,** said device comprising:
(i) two or three sensor elements, each composed of:
- an isolating support formed by a dielectric material; and
- a single capacitive transducer formed by interdigitated electrodes, wherein the finger width (3) of said interdigitated electrodes ranges from about 0.5 to about 10 µm;
wherein the interspace (2) extending between the fingers of said interdigitated electrodes has a width (3) ranging from about 0.5 to about 10 µm;
wherein the capacitive transducer has a surface area (7) of at least 4 mm²; wherein the materials of the electrodes or of the isolating support do not absorb water molecules;
(ii) a physical interface suitable for transducing a capacitive signal obtained from the sensor elements into an analog or digital signal;
wherein said physical interface has a sampling interval frequency of less than 500 Hz, or less than 200 Hz, or about or less than 100 Hz,
wherein said physical interface has a resolution of about or less than 0.1 pF, preferably about or less than 0.01 pF;
wherein the sensor elements and the physical interface are configured for determining a respiratory rate and breathing envelope; and
wherein the sensor elements are configured to discriminate and/or count water droplets suspended in said exhaled air stream as they deposit on the surface of the capacitive transducer.

## Patentansprüche

1. Verfahren zum Identifizieren von Atemveränderungen, die Atembeschwerden oder Atemwegserkrankungen repräsentieren, umfassend:
(a) Bereitstellen einer Vorrichtung, die umfasst:
(i) zwei oder drei Sensorelemente, die jeweils aus Folgendem bestehen:
- einer isolierenden Stütze, die durch ein dielektrisches Material gebildet wird; und
- einem einzelnen kapazitiven Wandler, der durch ineinandergreifende Elektroden gebildet wird,
wobei die Fingerbreite (3) der ineinandergreifenden Elektroden von etwa 0,5 bis etwa 10 µm reicht;
wobei der Zwischenraum (2), der sich zwischen den Fingern der ineinandergreifenden Elektroden erstreckt, eine Breite (3) aufweist, die von etwa 0,5 bis etwa 10 µm reicht;
wobei der kapazitive Wandler eine Oberflächenfläche (7) von mindestens 4 mm² aufweist;
wobei die Materialien der Elektroden oder der isolierenden Stütze keine Wassermoleküle absorbieren;
(ii) eine physische Schnittstelle, die zum Umsetzen eines kapazitiven Signals, das von den Sensorelementen erhalten wird, in ein analoges oder digitales Signal geeignet ist;
wobei die physische Schnittstelle eine Abtastintervallfrequenz von weniger als 500 Hz oder weniger als 200 Hz oder etwa oder weniger als 100 Hz aufweist, und
wobei die Schnittstelle eine Auflösung von etwa oder weniger als 0,1 pF, vorzugsweise etwa oder weniger als 0,01 pF aufweist;
(b) Platzieren der Vorrichtung, sodass eine Oberfläche (7) der Sensorelemente, die den kapazitiven Wandler umfassen, einem ausgeatmeten Luftstrom ausgesetzt wird und mit dem ausgeatmeten Luftstrom einen Winkel in einer Sagittalebene zwischen etwa -30° und etwa +30° bildet;
wobei die Sensorelemente und die physische Schnittstelle zum Bestimmen einer Atemfrequenz und Atemhülle konfiguriert sind; und
wobei die Sensorelemente konfiguriert sind, Wassertröpfchen, die in dem ausgeatmeten Luftstrom schweben, während sie sich auf der Oberfläche des kapazitiven Wandlers absetzen, zu unterscheiden und/oder zu zählen.

2. Verfahren nach Anspruch **1,** wobei der kapazitive Wandler eine Oberflächenfläche (7) von mindestens 9 mm² aufweist.

3. Verfahren nach Anspruch **1** oder **2,** wobei eine Oberfläche (7) der Sensorelemente, die den kapazitiven Wandler umfassen, einem ausgeatmeten Luftstrom ausgesetzt wird und mit dem ausgeatmeten Luftstrom einen Winkel in einer Koronalebene zwischen etwa -30° und etwa +30° bildet.

4. Verfahren nach einem der Ansprüche **1** bis **3,** wobei die Vorrichtung ferner umfasst:
(a) mindestens ein Temperatursensorelement, das zum Messen der Temperatur der Oberfläche (7) jedes Sensorelements, die dem Luftstrom ausgesetzt wird, angeordnet ist; und
(b) Mittel zum Erwärmen und/oder Kühlen der Oberfläche (7).

5. Verfahren nach Anspruch **4,** wobei das mindestens eine Temperatursensorelement aus der Gruppe ausgewählt wird, die aus einem Widerstand, einer Diode oder einem Thermoelement besteht.

6. Verfahren nach einem der Ansprüche **1** bis **5,** wobei die ineinandergreifenden Elektroden ein Metall umfassen, das aus der Gruppe ausgewählt wird, die Al, Ni, Au, Ag, Cu, Pt, Pd, Co und Fe oder Legierungen davon umfasst.

7. Verfahren nach einem der Ansprüche **1** bis **6,** wobei die Breite der Finger (1) der ineinandergreifenden Elektroden von 1 bis 5 µm reicht und vorzugsweise etwa 2 µm beträgt.

8. Verfahren nach einem der Ansprüche **1** bis **7,** wobei die Breite (3) der Zwischenräume (2), die sich zwischen den Fingern (1) der ineinandergreifenden Elektroden erstrecken, von 1 bis 5 µm reicht und vorzugsweise etwa 2 µm beträgt.

9. Verfahren nach einem der Ansprüche **1** bis **8,** wobei das dielektrische Material der isolierenden Stütze aus der Gruppe ausgewählt wird, die aus Keramik, Glas, Polymeren oder dergleichen besteht, und vorzugsweise Siliziumoxid ist.

10. Verfahren nach einem der Ansprüche **1** bis **9,** wobei die physische Schnittstelle aus der Gruppe ausgewählt wird, die einen Schmitt-Trigger, einen Ringoszillator, einen Sigma-Delta-Analog-Digital-Wandler (ADC), einen C-2C-Analog-Digital-Wandler und ein konstantes DC-Ladestromschema zur Kapazitätsmessung umfasst.

11. Verfahren nach einem der Ansprüche **1** bis **10,** wobei die physische Schnittstelle eine Abtastintervallperiode von weniger als 50 ms, bevorzugter von weniger als 20 ms und vorzugsweise von etwa 10 ms aufweist.

12. Verfahren nach einem der Ansprüche **1** bis **11,** wobei die physische Schnittstelle eine Auflösung von etwa oder weniger als 1 fF aufweist.

13. Verfahren nach einem der Ansprüche **1** bis **12,** wobei die Oberflächenfläche (7) des kapazitiven Wandlers gleich oder größer als 16 mm², und vorzugsweise gleich oder größer als 25 mm² ist.

14. Verfahren nach einem der Ansprüche **1** bis **13,** wobei die Vorrichtung ferner umfasst:
- Steuermittel zum Empfangen eines analogen oder digitalen Eingangssignals von der physischen Schnittstelle und zum Bereitstellen von Daten; und
- Mittel zum Aufzeichnen und/oder Übertragen und/oder Anzeigen von Daten, die durch das Steuermittel erzeugt werden.

15. Vorrichtung zum Implementieren des Verfahrens nach einem der Ansprüche 1 bis **14,** wobei die Vorrichtung umfasst:
(i) zwei oder drei Sensorelemente, die jeweils aus Folgendem bestehen:
- einer isolierenden Stütze, die durch ein dielektrisches Material gebildet wird; und
- einem einzelnen kapazitiven Wandler, der durch ineinandergreifende Elektroden gebildet wird,
wobei die Fingerbreite (3) der ineinandergreifenden Elektroden von etwa 0,5 bis etwa 10 µm reicht;
wobei der Zwischenraum (2), der sich zwischen den Fingern der ineinandergreifenden Elektroden erstreckt, eine Breite (3) aufweist, die von etwa 0,5 bis etwa 10 µm reicht; wobei der kapazitive Wandler eine Oberflächenfläche (7) von mindestens 4 mm² aufweist;
wobei die Materialien der Elektroden oder der isolierenden Stütze keine Wassermoleküle absorbieren;
(ii) eine physische Schnittstelle, die zum Umsetzen eines kapazitiven Signals, das von den Sensorelementen erhalten wird, in ein analoges oder digitales Signal geeignet ist;
wobei die physische Schnittstelle eine Abtastintervallfrequenz von weniger als 500 Hz oder weniger als 200 Hz oder etwa oder weniger als 100 Hz aufweist,
wobei die physische Schnittstelle eine Auflösung von etwa oder weniger als 0,1 pF, vorzugsweise etwa oder weniger als 0,01 pF aufweist;
wobei die Sensorelemente und die physische Schnittstelle zum Bestimmen einer Atemfrequenz und Atemhülle konfiguriert sind; und
wobei die Sensorelemente konfiguriert sind, Wassertröpfchen, die in dem ausgeatmeten Luftstrom schweben, während sie sich auf der Oberfläche des kapazitiven Wandlers absetzen, zu unterscheiden und/oder zu zählen.

## Revendications

1. Un procédé pour identifier des altérations de la respiration représentatives de troubles ou d'états respiratoires, comprenant :
(a) fournir d'un dispositif comprenant :
(i) deux ou trois éléments de détection, chacun composé de :
- un support isolant formé par un matériau diélectrique ; et
- un transducteur capacitif unique formé par des électrodes interdigitées, dans lequel la largeur de doigt (3) desdites électrodes interdigitées est comprise entre environ 0,5 et environ 10 µm ;
dans lequel l'espace intermédiaire (2) s'étendant entre les doigts desdites électrodes interdigitées a une largeur (3) comprise entre environ 0,5 et environ 10 µm ;
dans lequel le transducteur capacitif a une surface (7) d'au moins 4 mm² ;
dans lequel les matériaux des électrodes ou du support isolant n'absorbent pas les molécules d'eau ;
(ii) une interface physique convenant à la transduction d'un signal capacitif obtenu à partir des éléments de détection en un signal analogique ou numérique ;
dans lequel ladite interface physique a une fréquence d'intervalle d'échantillonnage inférieure à 500 Hz, ou inférieure à 200 Hz, ou environ ou inférieure à 100 Hz, et
dans lequel ladite interface a une résolution d'environ ou de moins de 0,1 pF, de préférence d'environ ou moins de 0,01 pF ;
(b) placer le dispositif de sorte qu'une surface (7) des éléments de détection comprenant ledit transducteur capacitif soit exposée à un courant d'air expiré et forme un angle, dans un plan sagittal, compris entre environ -30° et environ +30° avec ledit courant d'air expiré ;
dans lequel les éléments de détection et l'interface physique sont configurés pour déterminer une fréquence respiratoire et une enveloppe de respiration ; et
dans lequel les éléments de détection sont configurés pour discriminer et/ou compter les gouttelettes d'eau en suspension dans ledit courant d'air expiré lorsqu'elles se déposent sur la surface du transducteur capacitif.

2. Le procédé selon la revendication **1,** dans lequel le transducteur capacitif a une surface (7) d'au moins 9 mm².

3. Le procédé selon la revendication **1** ou **2,** dans lequel une surface (7) des éléments de détection comprenant ledit transducteur capacitif est exposée à un courant d'air expiré et forme un angle, dans un plan coronal, compris entre environ -30° et environ +30° avec ledit courant d'air expiré.

4. Le procédé selon l'une quelconque des revendications **1** à **3,** dans lequel le dispositif comprend en outre :
(a) au moins un élément de détection de température, disposé pour mesurer la température de ladite surface (7) de chaque élément de détection exposé audit flux d'air ; et
(b) des moyens pour chauffer et/ou refroidir ladite surface (7).

5. Le procédé selon la revendication **4,** dans lequel le au moins un élément de détection de température est choisi dans le groupe constitué par une résistance, une diode ou un thermocouple.

6. Le procédé selon l'une quelconque des revendications **1** à **5,** dans lequel lesdites électrodes interdigitées comprennent un métal choisi dans le groupe comprenant Al, Ni, Au, Ag, Cu, Pt, Pd, Co et Fe ou leurs alliages.

7. Le procédé selon l'une quelconque des revendications **1** à **6,** dans lequel la largeur desdits doigts (1) desdites électrodes interdigitées est comprise entre 1 et 5 µm et est de préférence d'environ 2 µm.

8. Le procédé selon l'une quelconque des revendications **1** à **7,** dans lequel la largeur (3) desdits espaces intermédiaires (2) s'étendant entre les doigts (1) desdites électrodes interdigitées est compris entre 1 et 5 µm et est de préférence d'environ 2 µm.

9. Le procédé selon l'une quelconque des revendications **1** à **8,** dans lequel le matériau diélectrique dudit support isolant est choisi dans le groupe constitué par la céramique, le verre, les polymères ou analogues, et est de préférence l'oxyde de silicium.

10. Le procédé selon l'une quelconque des revendications **1** à **9,** dans lequel ladite interface physique est choisie dans le groupe comprenant une bascule de Schmitt, un générateur de créneaux, un convertisseur analogique-numérique (CAN) deltasigma, un convertisseur analogique-numérique C-2C et un schéma de courant de charge continu constant pour la mesure de capacité.

11. Le procédé selon l'une quelconque des revendications **1** à **10,** dans lequel ladite interface physique présente une période d'intervalle d'échantillonnage inférieure à 50 ms, de préférence inférieure à 20 ms, et de préférence d'environ 10 ms.

12. Le procédé selon l'une quelconque des revendications **1** à **11,** dans lequel ladite interface physique présente une résolution d'environ ou inférieure à 1 fF.

13. Le procédé selon l'une quelconque des revendications **1** à **12,** dans lequel la surface (7) dudit transducteur capacitif est égale ou supérieure à 16 mm², et de préférence égale ou supérieure à 25 mm².

14. Le procédé selon l'une quelconque des revendications **1** à **13,** dans lequel le dispositif comprend en outre :
- des moyens de contrôle pour recevoir un signal analogique ou numérique d'entrée en provenance de ladite interface physique et pour fournir des données ; et
- des moyens pour enregistrer et/ou transmettre et/ou afficher des données générées par lesdits moyens de contrôle.

15. Un dispositif de mise en œuvre du procédé selon l'une quelconque des revendications **1** à **14,** ledit dispositif comprenant :
(i) deux ou trois éléments de détection, chacun composé de :
- un support isolant formé par un matériau diélectrique ; et
- un transducteur capacitif unique formé par des électrodes interdigitées, dans lequel la largeur des doigts (3) desdites électrodes interdigitées est comprise entre environ 0,5 et environ 10 µm ;
dans lequel l'espace intermédiaire (2) s'étendant entre les doigts desdites électrodes interdigitées a une largeur (3) comprise entre environ 0,5 et environ 10 µm ;
dans lequel le transducteur capacitif a une surface (7) d'au moins 4 mm² ;
dans lequel les matériaux des électrodes ou du support isolant n'absorbent pas les molécules d'eau ; et
(ii) une interface physique convenant à la transduction d'un signal capacitif obtenu à partir des éléments de détection en un signal analogique ou numérique ;
dans lequel ladite interface physique a une fréquence d'intervalle d'échantillonnage inférieure à 500 Hz, ou inférieure à 200 Hz, ou environ ou inférieure à 100 Hz,
dans lequel ladite interface physique a une résolution d'environ ou moins de 0,1 pF, de préférence d'environ ou moins de 0,01 pF ;
dans lequel les éléments de détection et l'interface physique sont configurés pour déterminer une fréquence respiratoire et une enveloppe de respiration ; et
dans lequel les éléments de détection sont configurés pour discriminer et/ou compter les gouttelettes d'eau en suspension dans ledit courant d'air expiré lorsqu'elles se déposent sur la surface du transducteur capacitif.
